# EUROPEAN PATENT APPLICATION

(11) **EP 3 211 073 A1**
(43) Date of publication of application: **30.08.2017**
(21) Application number: 15852404.1
(22) Date of filing: 19.10.2015
(51) Int. Cl.: C12N 7/01, A61K 39/12, A61P 31/18

(54) **REPLICATIVE VACCINIA VIRUS VECTOR HIV VACCINE**

(30) Priority: 20.10.2014 CN 201410558649
(71) Applicant: National Center for Aids/STD Control and Prevention, Chinese Center for Disease Control and Prevention, Beijing 102206 (CN)
(72) Inventor: SHAO, Yiming, Beijing 102206 (CN); ZHANG, Qicheng, Beijing 102206 (CN); LIU, Ying, Beijing 102206 (CN)
(74) Representative: Nederlandsch Octrooibureau
(86) International application number: PCT/CN2015/092170
(87) International publication number: WO 2016/062226

(57) **Abstract**

Provided is a recombinant replicative vaccinia virus, the virus comprising a polynucleotide encoding HSV-TK and HIV antigens. Also provided is a live vector HIV vaccine composition comprising the recombinant replicative vaccinia virus.

## Description

### Technical Field

The present invention relates to the field of medical biotechnology, and specifically relates to an AIDS vaccine of replicative vaccinia virus, which can induce a high level of humoral and cellular immune responses against human immunodeficiency virus and allow an effective control of severe adverse effects that might be caused by the vaccine.

### Technical Background

Vaccinia virus vector is one of the most intensively and extensively studied viral vectors, which can be used as a vector for constructing vaccines. Non-replicative vaccinia virus vectors are safe, but have a relatively weaker immunogenicity. In particular, the immune response they induced in human is distinctly weaker than in monkey. Replicative vaccinia virus vectors have strong immunogenicity, and can induce long lasting immunity, but they have potential safety issues such as causing severe adverse effects.

Because smallpox had been eliminated for many years, vaccinia virus as a smallpox vaccine has no longer been inoculated for more than 30 years, and as a result, people rarely pay attentions to developing drugs against vaccinia virus. With the application of replicative vaccinia virus vectors in the field of vaccine development, it is desired to develop medicines and measures that can be used as effective treatments when severe adverse effects emerge.

### Summary of the Invention

In a first aspect, the present invention provides a recombinant replicative vaccinia virus, in its TK region comprising a first polynucleotide encoding an HSV-TK and a second polynucleotide encoding an HIV antigen.

Preferably, the recombinant replicative vaccinia virus of the invention is a vaccinia virus Tiantan strain.

In one specific embodiment, the first polynucleotide in the recombinant replicative vaccinia virus of the invention comprises a nucleotide sequence set forth in SEQ ID NO: 3. In another embodiment, the first polynucleotide in the recombinant replicative vaccinia virus of the invention comprises a nucleotide sequence which is at least 80%, at least 85%, at least 90%, at least 95%, or at least 99% identical to the nucleotide sequence set forth in SEQ ID NO: 3, and wherein the first polynucleotide encodes a thymidine kinase (TK)from HSV-1.

In another specific embodiment, the second polynucleotide in the recombinant replicative vaccinia virus of the invention encodes a gp145 2M derived from 97CN001 which is the predominant HIV epidemic strain in China, and comprises a nucleotide sequence set forth in SEQ ID NO:1. In another embodiment, the second polynucleotide in the recombinant replicative vaccinia virus of the invention encodes a gp145 2M derived from 97CN001, which is the predominant HIV epidemic strain in China, or an immunogenic fragment thereof In another embodiment, the second polynucleotide in the recombinant replicative vaccinia virus of the invention comprises a nucleotide sequence which is at least 80%, at least 85%, at least 90%, at least 95%, or at least 99% identical to the nucleotide sequence set forth in SEQ ID NO:1, and wherein the second polynucleotide encodes a gp145 2M from HIV-1.

Preferably, the recombinant replicative vaccinia virus of the invention also comprises a third polynucleotide encoding an additional HIV antigen. In one preferred embodiment, the third polynucleotide is inserted into the HA region of the replicative vaccinia virus. In one specific embodiment, the third polynucleotide encodes a gag derived from 97CN001, which is the predominant HIV epidemic strain in China, and comprises a nucleotide sequence set forth in SEQ ID NO:2. In another embodiment, the third polynucleotide encodes a gag derived from 97CN001 which is the predominant HIV epidemic strain in China, or an immunogenic fragment thereof. In another embodiment, the third polynucleotide comprises a nucleotide sequence which is at least 80%, at least 85%, at least 90%, at least 95%, or at least 99% identical to the nucleotide sequence set forth in SEQ ID NO:2, and the third polynucleotide encodes a gag from HIV-1.

In one preferred embodiment, the present invention provides a recombinant replicative vaccinia virus, which is a vaccinia virus Tiantan strain comprising a gene encoding gp145 2M derived from 97CN001 which is the predominant HIV epidemic strain in China, and a gene encoding an HSV-TK, both genes being inserted into the TK region of the vaccinia virus Tiantan strain, and further comprising a gene encoding a gag derived from 97CN001 inserted into the HA region of the vaccinia virus Tiantan strain.

The invention further provides a composition of a live vector HIV vaccine, which comprises the recombinant replicative vaccinia virus of the invention mentioned in the first aspect and a pharmaceutically acceptable carrier and/or adjuvant.

The present invention also relates to a method for preventing and/or treating HIV infection in a subject, which comprises administrating the composition of the live vector HIV vaccine comprising the recombinant replicative vaccinia virus of the invention mentioned in the first aspect to the subject. The present invention also relates to the use of the recombinant replicative vaccinia virus of the invention mentioned in the first aspect in the preparation of a composition of a live vector HIV vaccine for preventing and/or treating HIV infection in a subject. Preferably, said subject is an immunocompromised patient, such as one having congenital immunodeficiency, HIV infection, or is undergoing immunosuppressive therapy.

The present invention also provides an AIDS immunization kit, and said kit comprises a plurality of components and an instruction indicating an immunization procedure, wherein one of the components is the composition of the live vector vaccine of the invention.

### Description of the Drawings

Figure 1 depicts the *in vitro* inhibition effects of GCV against the recombinant vaccinia virus.
Figure 2 depicts the inhibition effects of GCV against TT-TK and TT-EnvTK replications in the brains of mice.
Figure 3 depicts the inhibition effects of GCV against TT-TK replication in the ovaries of mice.
Figure 4 depicts immune responses in mice induced by TT-EnvTK; A: cellular immunity; B: humoral immunity.
Figure 5 depicts the immune interference among several HIV antigens.

### Deposition Information

The recombinant vaccinia virus TT-TK was deposited on October 20, 2014, in China General Microbiological Culture Collection Center (CGMCC), under the deposition No.: CGMCC No. 9810.

The recombinant vaccinia virus TT-EnvTK was deposited on October 20, 2014, in China General Microbiological Culture Collection Center (CGMCC), under the deposition No.: CGMCC No. 9808.

The recombinant vaccinia virus TT-gag was deposited on October 20, 2014, in China General Microbiological Culture Collection Center (CGMCC), under the deposition No.: CGMCC No. 9809.

### Sequence Information

SEQ ID NO:1: Gp 145 2M gene sequence, which is originated from HIV-1;
SEQ ID NO:2: Gag gene sequence, which is originated from HIV-1;
SEQ ID NO:3: HSV-TK gene sequence, which is originated from HSV-1;
SEQ ID NO:4: plasmid pSC65-DR-GN gene sequence.

### Detailed Description of the Invention

Acquired Immunodeficiency Syndrome, (AIDS) is a transmissible disease caused by an infection of Human Immunodeficiency Virus (HIV). Since the first case discovered in 1981, AIDS has been spread worldwide at an alarming rate, and becomes one of the most serious viral diseases threatening the human lives and health. The epidemicity of AIDS brings huge influences to the social and economic developments of the world. In some developing countries, HIV infections have resulted in shortenings of average lifespan, reductions of labor, and food shortages, which causing regressions in social and economic developments for 20 years. The epidemicity of HIV in China went through three stages: importing stage (1985-1988), spreading stage (1989-1994), and growing stage (1995-now).

In recent years, people gained great progresses in the researches and applications of anti-HIV medicines, and some of the infected patients received relatively good treatments. However, the emergence of medicine resistant strains causes the medicine not effective for every patient, the complicated administration procedure makes some of the patients unable to keep on taking medicines on time and thus result in failures of treatments, and expensive costs also make the treatments difficult to be widely popularized. The historical experiences demonstrated that the most cost-effective way to control the epidemicity of a disease is to use vaccines, successful examples including smallpox, poliomyelitis, measles, hepatitis, and so on. Accordingly, the development for a safe and effective AIDS vaccine is always the objective of scientific researchers.

The scientists in various countries all agree that an effective AIDS vaccine must be able to induce the generation of HIV specific CD8+ cytotoxic T-lymphocyte responses (CTL) and neutralizing antibody responses in an organism. It is difficult for current single vaccines to achieve such an objective, and a combined immunization using different types of vaccines is required, which will increase the immune-effects of the AIDS vaccines. Candidate vaccines can be chosen from the followings: traditional vaccines (inactivated vaccine and attenuated live vaccine), synthetic peptide and protein subunit vaccine, DNA vaccine, and live vector vaccine.

Compared to other types of vaccines, the advantages of live vector vaccines are in that: (1) they can actively infect target tissues or cells, and thus increase the efficiency of exogenous genes entering the cells; (2) vectors *per se* possesses an adjuvent effect, and can induce the generation of cytokines and chemokines; (3) most of them can induce immune responses for a long period of time.

Researches on using vaccinia virus as vectors for live vector vaccines are most profound and extensive, but most of vaccines entered clinical trial phase use non-replicative vectors, such as MVA, NYVAC, and ALVAC. However, the clinical trials show that the immunogenicity of non-replicative vectors is relatively weak, and the same vaccine can only induce distinctly weaker immune responses in human than in monkey. This might be related to the non-replicative feature thereof. After infecting host cells, non-replicative vector vaccine can only undergo one cycle of antigen expression, processing and presenting procedures, due to the fact that it cannot generate infectious virus and initiate a new cycle of infection, and thus the stimulation for immune system is only limited and short-acting. For this reason, the international researching focus for live vector vaccines has turned from non-replicative types to replicative types, so as to fully exert the replicative property of live vectors, and better stimulate the organisms to generate immune responses.

The replicative vaccinia virus vector has strong immunogenicity, and can induce long lasting immunity, but it has potential safety issues such as causing severe adverse effects. Especially for those individuals who are immunocompromized due to congenital immunodeficiency, immunosuppressive therapy, HIV infection, or so on, replicative vaccinia virus vector vaccines might trigger severe adverse effects [1,2].

Because smallpox has been eliminated for many years, vaccinia virus as a smallpox vaccine has no longer been inoculated for more than 30 years, and thus people rarely pay attentions to developing drugs against vaccinia virus. With the application of replicative vaccinia virus vector in the field of vaccine development, it is desired to develop medicines and measures that can be used as effective treatments when severe adverse effects emerge.

Suicide gene system is a method widely used for treating diseases like cancer in recent years. Conventional combinations of suicide gene/medicine include: cytosine deaminase (CD)/5-fluorocytosine^{[3]}, nitroreductase/CB 1954^{[4]}, thymidine phosphorylase (TP)/5'-deoxy-5-fluorouridine (5'-DFUR)^{[5]}, purine nucleoside phosphorylase (PNP)/6-methylpurine-2'- deoxyriboside (MeP-dR)^{[6]}, and herpes simplex virus thymidine kinase (HSV-TK)/ganciclovir (GCV)^{[7]}, wherein HSV-TK/GCV is the suicide gene system that mostly researched and applied.

GCV activity is specifically depends on the TK gene of the HSV. The HSV TK can phosphorylate GCV, and generate a monophosphorylated form thereof (GCV-MP)^{[8]}. Subsequently cell kinase further phosphorylates the GCV-MP, and generates a diphosphate form (GCV-DP) and a toxic triphosphate form (GCV-TP). GCV-TP is a DNA polymerase inhibitor, which can inhibit the replication of DNA by competitively inhibiting the combination of deoxyguanosine triphosphate and DNA polymerase, or by inserting into a newly-generated chain to inhibit DNA replication, and resulted in a termination of the extension of the newly-generated chain, and ultimately kill the cells ^{[9]}. Since GCV cannot be directly phosphorylated by the mammalian cell kinase, the prodrug only has selective toxicity against cells expressing the HSV-TK, with minor adverse effects.

Currently it has been reported that viral vectors carrying an HSV-TK/GCV suicide gene system are used for treating tumors, wherein the function of the HSV-TK/GCV suicide gene system is to directly kill the target cells, rather than to ensure the usage safety of the live virus vectors during the application procedure and control the possible adverse effects related to the vaccine vectors. Also, current HSV-TK/GCV suicide gene system is mainly used for adenovirus vectors and adeno-associated virus vectors, and the compatibility between the HSV-TK/GCV system and the vaccinia virus vector, especially the vaccinia virus Tiantan strain as the replicative vaccinia virus vector, has not yet been tested. The TK gene of vaccinia virus only has about 16% of homology to HSV-TK, and it seems that Ganciclovir has no inhibition effect on vaccinia virus (EC50>300 µM)^{[10]}.

The inventors introduced the HSV-TK gene into a replicative vaccinia virus vector, so as to provide a safe and controllable replicative vaccinia virus vector AIDS vaccine. The inventors demonstrated that the introduction of HSV-TK gene into the replicative vaccinia virus AIDS vaccine effectively controlled severe adverse effects that might be caused by the replicative vaccinia virus vector, increased the safety of the vaccine, and solved the safety issue caused by the replicative vaccinia virus vector without affecting the immunogenicity of the replicative poxvirus vector. The replicative vaccinia virus vector AIDS vaccine of the invention is particularly suitable for immunocompromised patients, such as patients having congenital immunodeficiency, HIV infection, or patients undergoing immunosuppressive therapies.

With respect to AIDS vaccines, the selection of HIV antigens is also very important. The HIV-specific function of eliminating CD8+ T-cells (CTL) plays an important role in HIV replications. Gag-specific CTL is advantageous for controlling virus replications in infected patients. Env-induced neutralizing antibody can neutralize HIV. The titre of V1V2 binding antibody is positively correlated with the immune protection of the vaccine. Therefore several antigens should be selected for an AIDS vaccine to induce different types of immune responses. Moreover, the inventors discovered that, with the prerequisite that the expression of several antigens is assured, the respective introductions of HIV-1 gag gene and gp145 2M gene into the HA region and the TK region of the replicative vaccinia virus vector can also avoid the interference between different antigens.

Accordingly, in a first aspect, the invention provides a recombinant replicative vaccinia virus comprising, in its TK region, a first polynucleotide encoding HSV-TK and a second polynucleotide encoding HIV antigen.

Preferably, the recombinant replicative vaccinia virus of the invention is a vaccinia virus Tiantan strain.

In one specific embodiment, the first polynucleotide in the recombinant replicative vaccinia virus of the invention comprises the nucleotide sequence set forth in SEQ ID NO:3. In another embodiment, the first polynucleotide in the recombinant replicative vaccinia virus of the invention comprises a nucleotide sequence which is at least 80%, at least 85%, at least 90%, at least 95%, or at least 99% identical to the nucleotide sequence set forth in SEQ ID NO:3, and the first polynucleotide encodes a TK from HIV-1.

In another specific embodiment, the second polynucleotide in the recombinant replicative vaccinia virus of the invention encodes a gp145 2M derived from 97CN001, the predominant HIV epidemic strain in China, and comprises the nucleotide sequence set forth in SEQ ID NO:1. In another embodiment, the second polynucleotide in the recombinant replicative vaccinia virus of the invention encodes a gp145 2M derived from 97CN001, the predominant HIV epidemic strain in China, or an immunogenic fragment thereof. In another embodiment, the second polynucleotide in the recombinant replicative vaccinia virus of the invention comprises a nucleotide sequence which is at least 80%, at least 85%, at least 90%, at least 95%, or at least 99% identical to the nucleotide sequence set forth in SEQ ID NO:1, and the second polynucleotide encodes a gp145 2M from HIV-1.

Preferably, the recombinant replicative vaccinia virus of the invention further comprises a third polynucleotide encoding an additional HIV antigen. In one preferred embodiment, the third polynucleotide is inserted into the HA region of the replicative vaccinia virus. In one specific embodiment, the third polynucleotide encodes a gag derived from 97CN001, the predominant HIV epidemic strain in China, and comprises the nucleotide sequence set forth in SEQ ID NO:2. In another embodiment, the third polynucleotide encodes a gag derived from 97CN001, the predominant HIV epidemic strain in China, or an immunogenic fragment thereof. In another embodiment, the third polynucleotide comprises a nucleotide sequence which is at least 80%, at least 85%, at least 90%, at least 95%, or at least 99% identical to the nucleotide sequence set forth in SEQ ID NO:2, and the third polynucleotide encodes a gag from HIV-1.

In one preferred embodiment, the invention provides a recombinant replicative vaccinia virus, which is a vaccinia virus Tiantan strain which comprises a gene encoding a gp145 2M derived from 97CN001, the predominant HIV epidemic strain in China, and a gene encoding HSV-TK, both being inserted into the TK region of the vaccinia virus Tiantan strain, and further comprises a gene encoding a gag derived from 97 CN001 inserted into the HA region of the vaccinia virus Tiantan strain.

The invention further provides a composition of live vector HIV vaccine, which comprises the recombinant replicative vaccinia virus of the invention described in the first aspect, and a pharmaceutically acceptable carrier and/or adjuvant.

The present invention also relates to a method for preventing and/or treating HIV infection in a subject, which comprises administrating to the subject the composition of live vector HIV vaccine that comprises the recombinant replicative vaccinia virus of the invention mentioned in the first aspect. The present invention also relates to use of the recombinant replicative vaccinia virus of the invention mentioned in the first aspect in the preparation of a composition of live vector HIV vaccine for preventing and/or treating HIA infections in a subject. Preferably, said subject is an immunocompromised patient, such as having congenital immunodeficiency, HIV infection, or undergoing immunosuppressive therapies.

The present invention also provides an AIDS immunization kit, which comprises a plurality of components and an instruction indicating the immunization procedure, wherein one of the components is the composition of the live vector vaccine of the invention.

### Examples

### Example 1: The construction of transfer plasmids for the recombinant vaccinia virus that expresses HSV-TK gene

The process for constructing the transfer plasmids pVI75-GFP-TK and pVI75-GFP-EnvTK is as following:
1. The construction of plasmid pSC65-TK1
   The genomic DNA of herpes simplex virus HSV-1 was used as the template, primers HSV-TK-EcoRI/XhoI-up (5' CAA***GAATTCCTCGAG***TCAGTGGTGGTGGTGGTGGTG GTTAGCCTCCCCC 3', SEQ ID NO:10) and HSV-TK-SalI-for (5' TAC***GTCGAC*** ATGGCTTCGTACCCCTGCCATCAGC 3', SEQ ID NO:11) carrying *Xba*I and *Sal*I restriction sites and 3'-end His-tag were used for amplifying HSV-TK gene by PCR. The amplification conditions were as follows: 95°C 5 min; 95°C 30s, 55°C 30s, 72°C 1.5 min, 5 cycles; 95°C 30s, 60°C 30s, 72°C 1.5 min, 25 cycles; 72°C 5 min. The size of the HSV-TK fragment is 1149 bp.
   The HSV-TK fragment obtained through PCR amplification was double-digested by enzymes *Sal*I and *EcoR*I and then ligated into the plasmid pSC65 double-digested by the same enzymes (deposited on Feburary 24, 2004, in China General Microbiological Culture Collection Center (CGMCC), under the deposition No. CGMCC No. 1097), and obtained the pSC65-TK1 plasmid. The pSC65-TK1 plasmid was identified by *Sal*I and *EcoR*I double-digestion resulting two fragments of 1351 bp and 4051 bp. The TK gene was sequenced, and the result was shown in SEQ ID NO:3.
2. The construction of the plasmid pSC65-TK1-gp145 2M
   In order to construct the recombinant plasmid pSC65-TK1-gp145 2M that simultaneously expresses HSV-TK and HIV-1gp145 2M, the plasmid pDRVISV1452M (the *E. coli* containing the plasmid was deposited on May 22, 2008, in CGMCC, under the deposition No. CGMCC No. 2513) was used as the template, and primers gp145 2M for-Sal (5' ACGC***GTCGAC***AGAGATATCGACACCATGG ACAGGG 3', SEQ ID NO:12) and gp145 2M -rev-PacI (5' GCC***TTAATTAA***TCAGTAGCCCTGCCTCACCCTGTTC 3', SEQ ID NO:13) were used to amplify the HIV-1 gyp145 2M gene of 2.1kb in size by PCR. The conditions for the PCR reaction were: 95°C pre-denaturation for 5 min; 95°C 30s, 60°C 30s, 72°C 2.5 min, altogether 30 cycles; 72°C sufficient extension for 5 min. The HIV-1 gp145 2M fragment obtained by the amplification and the plasmid pSC65-TK1 were double-digested by *Sal*I and *Pac*I and then ligated to obtain the plasmid pSC65-TK1-gp145 2M. Identification was performed by double-digestion with *Pac*I and *Xho*I resulting two fragments of 4076 bp and 3310 bp.
3. The construction of the transfer plasmids pTK-gp1452M and pTK
   The plasmids pSC65-TK1-gp145 2M and pSC65-DR-GN (the sequence of the plasmids can be seen in SEQ ID NO:4) were double-digested with *Pac*I and *Xho*I and then ligated, resulting the transfer plasmid pTK-gp1452M. Double-digestion of the transfer plasmid pTK-gp1452M with *Pac*I and *Xho*I results two fragments of 3461 bp and 6231 bp.
   The plasmids pSC65-TK1 and pSC65-DR-GN were double-digested with *Pac*I and *Xho*I and then ligated, resulting the transfer plasmid pTK. Primers HSV-TK-EcoRI/XhoI-up (SEQ ID NO:10) and HSV-TK-SalI-for (SEQ ID NO:11) were adopted for PCR amplification, and the product was a fragment of about 1.2kb in size.

### Example 2: Construction and characterization of the recombinant vaccinia virus

The TKL and TKR regions of the transfer plasmids pTK and pTK-gp1452M were homologously recombined with the vaccinia virus Tiantan strain, allowing the target genes HSV-*TK* and *GP145 2M* together with marker genes *neo* and *GFP* to be recombined into the TK sequence of the vaccinia virus genome. In the presence of G418, homologous recombination did not take place within the molecules due to the selective pressure, marker genes *neo* and *GFP* temporally remained in the genome. The recombinant vaccinia virus that not only contains the target gene but also possesses green fluorescence was selected under fluorescence microscope (the growth of virus in which no recombination took place was inhibited due to the presence of G418). Subsequent selection was conducted under the conditions without G418, the recombinant vaccinia virus with green fluorescence *per se* can undergo an intramolecular secondary homologous recombination between an intact *TKR* and a small section of about 200 bp of a *TKR-DR* fragment brought in by the transfer plasmid. Therefore, the *neo* gene and *GFP* gene are lost. The recombinant vaccinia virus that only contains the target gene was obtained by selection of recombinant virus without green fluorescence.

### 1. Construction and purification of the recombinant vaccinia virus

Chicken embryo fibroblasts (CEFs) of 80-90% confluency were infected with a vaccinia virus Tiantan strain VTT (the virus was provided by National Vaccine and Serum Institute of Beijing) at a viral exposure amount of MOI=1, and cultivated at 37°C, 5% CO₂ for 2 hours. Using liposome to respectively transfect the recombinant plasmids pTK and pTK-gp145 2M into the cells, and the process is delineated in the instruction manual of the kit (Invitrogen, Lipofectamine® 2000 transfection Reagent, Cat. 11668-027). The recombinant plasmids underwent homologous recombination with VTT in the cells, and simultaneously resulted in losses of the VTT thymidine kinase gene. 24 hours after the transfection, the cell sample was collected and frozen-thawed for 3 times.

CEF cells in confluency was first cultured using the Eagle's medium containing 0.4mg/mL of G418 for 24h, 300µl of the collected transfection virus liquid was inoculated to CEF cells pre-treated by 0.4mg/mL of G418 for 24h. The Eagle's medium still contained 0.4mg/mL of G418 and 1% of low melting-point agarose. After being cultured at 37°C for 48 hours, a plaque with green fluorescence was picked and inoculated into 1mL of the Eagle's maintenance medium. After being frozen-thawed for 3 times, 100µl of the medium was inoculated to CEF cells that have been pre-treated by G418. By repeating the above procedures, single plaques were continuously purified for 2 generations, and the third generation of plaque with green fluorescence was thus obtained. The virus picked at this point contained gp145 2M, TK, GFP and *neo* genes. After frozen-thawing the third generation of plaque with green fluorescence for 3 times, 100µl of each sample was respectively picked to infect CEF cells without G418 treatment. The cells were cultivated at 37°C for 48 hours, and then white plaques without fluorescence were selected. After freeze-thawing the white plaque virus for 3 times, 100µl of sample was then picked to inoculate CEF cells pre-treated by G418. By repeating the above procedure and continuously purifying single plaques for 5 generations, the purified recombinant vaccinia viruses TT-TK and TT-EnvTK were obtained. TT-TK and TT-EnvTK were deposited on October 20, 2014, in China General Microbiological Culture Collection Center (CGMCC), under the deposition No.: TT-TK: CGMCC No. 9810, TT-EnvTK: CGMCC No. 9808, respectively.

### 2. PCR identification of the recombinant virus

The genomic DNA of recombinant viruses TT-TK and TT-EnvTK were extracted, and identification primers tkL-up (TAACGTGATGGATATATTAAAGTCG, SEQ ID NO:14) and tkR-low (AACGACACAACATCCATTTTTAAG, SEQ ID NO:15) were used to perform a PCR identification. The results showed that the sizes of the fragments using VTT, TT-TK, and TT-EnvTK as templates were 674 bp, 2830bp, and 4966bp respectively, indicating that both gp145 2M and TK genes were correctly recombined into the VTT genome.

### 3. Expression of target gene of the recombinant vaccinia virus

The recombinant vaccinia viruses TT-TK, TT-EnvTK, VTT, and VTKgpe (deposited in China General Microbiological Culture Collection Center, under the deposition No.: CGMCC No.1099) were respectively used at a dose of 1PFU/cell to infect CEF cells. Cells were collected after 24 hours cultivation, 1ml of protein extraction buffer (1% SDS, 1mmol/L PMSF, 20mmol/L Tris-Cl pH7.0, 1% β-mercaptoethanol) was added, and immediately blended, and repeatedly frozen-thawed for 3 times. PAGE electrophoresis and membrane transfer were performed, and 5% skim milk was used for blocking at room temperature for 2 hours. Mouse anti-His-Tag antibody (Gibco, Cat. Fk0168) was used to detect the expression of HSV-TK, and HIV-1 SF2 GP160 (from NIH AIDS Research & Reference Reagent Program) anti-serum was used to detect the expression of HIV-Env (1:1000 diluted). The membranes were incubated with primary antibodies diluted in 5% skim milk at the room temperature for 2 hours, and washed with PBS for 3 times, 10 minutes for each time. Corresponding secondary antibodies labeled by horseradish peroxidase diluted at 1:5000 in 5% skim milk were added and incubated at the room temperature for 1 hour. The membranes were washed with PBS for 3 times, 10 min for each time. After ECL color development, the results showed that, TT-TK and TT-EnvTK had a band of about 40 kD, which was the same size as HSV-TK-His, indicating that the recombinant virus strain TT-TK can express HSV-TK gene. An HIV-Env band of about 140 kD was detected in both TT-EnvTK and the positive control, indicating that the recombinant virus strain TT-EnvTK can express the HIV-Env gene.

### Example 3: The inhibition effect of Ganciclovir (GCV) against the recombinant vaccinia virus

### 1. The in vitro inhibition experiment of GCV for the recombinant vaccinia viruses

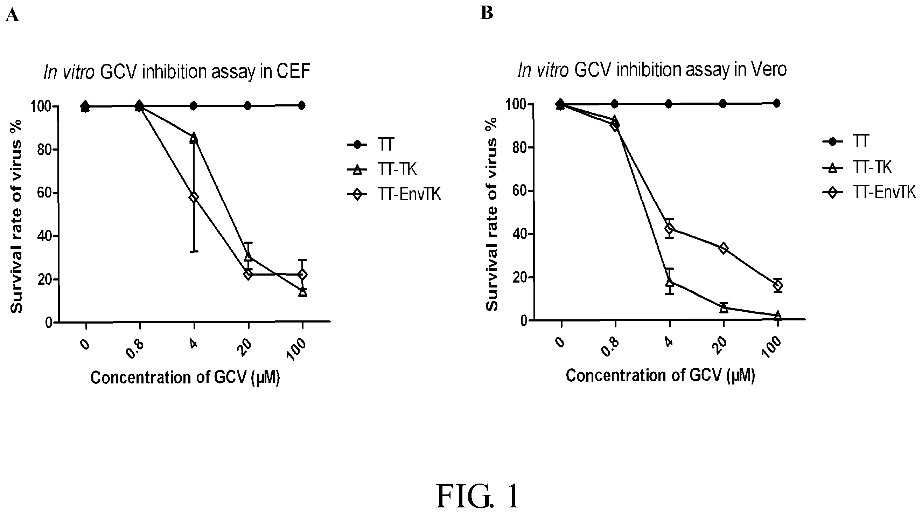

VTT, TT-TK, and TT-TKEnv were respectively inoculated at a virus exposure amount of about 100 PFU/well to infect a single layer of CEF or Vero cells in a 6-well plate. After infection at 37°C, 5% CO₂ for 2 hours, a medium containing a final concentration of 0∼100µM of GCV was then added to the cells. After continuously cultivated for 48 hours, the number of plaques formed in each well was counted, and inhibition curves of GCV against each of the recombinant vaccinia viruses were plotted. The results are shown in Figure 1. In CEF (A) and Vero (B), GCV showed clear inhibition effects on TT-TK and TT-EnvTK, and the virus plaque numbers for both of TT-TK and TT-EnvTK were negatively correlated to the concentration of GCV. GCV showed no inhibition effects on VTT.

### 2. The in vivo inhibition experiment of GCV for the recombinant vaccinia viruses

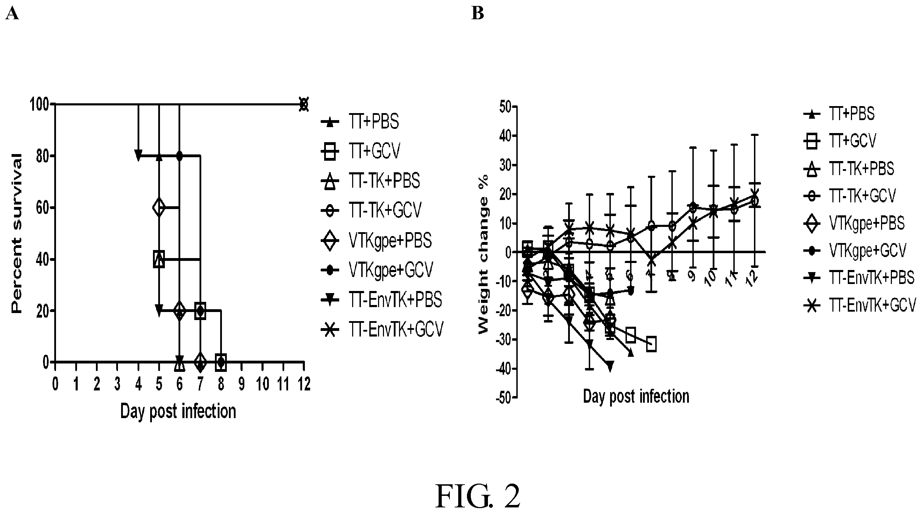

Three-week old BALB/c mice were randomly divided into different groups, which were inoculated in brain with 10×LD₅₀ doses of VTT, TT-T, or TT-EnvTK, and then were injected with a dose of 80 mg/kg/day of GCV. PBS was set as a control group. The changes of body weight and mortality of mice in each group were recorded every day. The results are showed in Figure 2. During the experiment, the mice in PBS control groups began to show decreases in body weights and activities 3 days after the virus challenge. All the animals in each control group died on day 7. The body weight loss and the mortality of the VTT-mice (mice inoculated with VTT) in GCV injected group were similar to the PBS control group. All the animals died on day 8. The body weights of the TT-TK-mice and TT-EnvTK-mice (inoculated with TT-TK and TT-EnvTK) in GCV injected groups slightly decreased, and gradually recovered since day 3. All the animals survived. This result demonstrates that GCV can inhibit the replication of TT-TK and TT-EnvTK in the brain of mice.

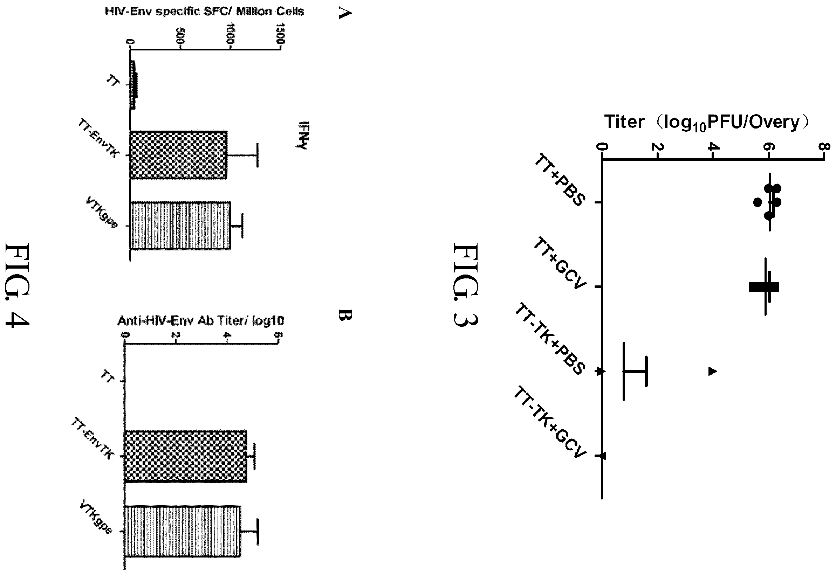

Eight-week old female C57BL mice were randomly divided into different groups. Each was injected in the abdominal cavity using 10⁷ PFU doses of VTT and TT-TK. Mice were treated with abdominal injection of GCV in a dose of 80 mg/kg/day and PBS was set as control groups. The mice in each group were sacrificed 8 days after the virus challenge and the ovaries on both sides were removed. The viral titre therein was titrated on CEFs. The results are shown in Figure 3. Injected with PBS or GVC after VTT inoculation, the virus titres in mice ovaries were up to 1.2×10⁶ PFU and 1×10⁶ PFU, respectively. Injected with PBS after TT-TK inoculation, the virus titre in mice ovaries was 2×10³ PFU. No virus was detected in mice ovaries that were injected with GCV. This result indicated that GCV can inhibit the replication of TT-TK in mice ovary.

### Example 4: The immunogenicity of the recombinant vaccinia virus

Six-week old female BALB/c mice were randomly divided into different groups, with 5 mice in each group. DNA vaccines of 50µg/mouse/dose were intramuscularly injected at week 0, 3, and 6, and at week 9, 10⁷ PFU of TT-EnvTK, as well as control virus strains VTKgpe and VTT, were injected respectively as boosters. One week after the injections of the boosters, the mice were sacrificed, serum was isolated, and splenic lymphocytes were prepared. ELISA process was used to detect HIV-specific antibody, the ELISPOT of IFN-γ was used to detect cellular immune responses. The results showed that, the strength of the specific T-cell immune responses induced by the recombinant virus TT-EnvTK against HIV-Env is 956 SFC/10⁶ splenic cells, and that induced by the control vaccine strain VTKgpe is 996 SFC/10⁶ splenic cells. Statistical analysis showed that, there was no significant difference between the two groups (P>0.05) (Figure 4A). The antibody titre induced by TT-EnvTK was 10^{4.7}, and that induced by VTKgpe was 10^{4.5}. Statistical analysis showed that, there was no significant difference between the two groups (P>0.05) (Figure 4B). The results showed that, TT-EnvTK can induce HIV-specific cellular and humoral immune responses, and the strength of immune responses showed no significant difference with the control virus.

### Example 5: The interference among several HIV antigen genes in the same recombination region

The expression of several HIV antigen genes in the same recombination region can simplify the virus construction procedure, but there seemed to be certain interference among the exogenous genes. Regarding this, the inventors compared the gag specific cellular immune responses induced by recombinant viruses TT-gag and VTKgpe.

The TK region of VTKgpe simultaneously expressed gag, pol, and env genes of HIV, while the recombinant vaccinia virus TT-gag (deposited on October 20, 2014, in China General Microbiological Culture Collection Center (CGMCC), under the deposition No.: CGMCC No. 9809) only expressed gag gene.

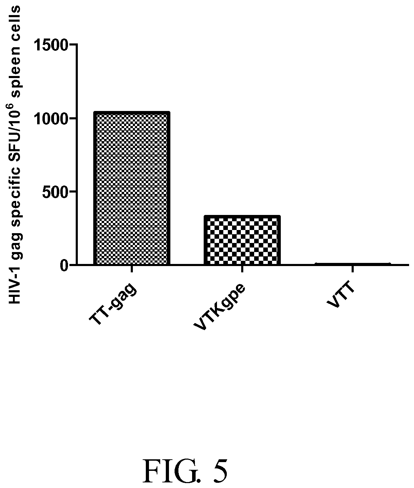

Six-week old female BALB/c mice were randomly divided into different groups, with 5 mice in each group. DNA vaccines of 50µg/mouse/dose were intramuscularly injected at week 0, 3, and 6, and at week 9, VTKgpe, TT-gag, and control virus VTT were injected as boosters respectively. One week after the injections of the boosters, the mice were sacrificed, serum was isolated, and splenic lymphocytes were prepared. The ELISPOT results of IFN-γ showed that, the strength of the specific T-cell immune responses against HIV-1 gag induced by the recombinant virus VTKgpe was 330 SFC/10⁶ splenic cells, which was significantly lower than the 1038 SFC/10⁶ splenic cells induced by TT-gag (Figure 5). Therefore, according to the result, the interference among the several HIV antigen genes in the same recombination region indeed existed, which decreased the strength of immune responses. Therefore, HIVgag, gp145 2M genes should be inserted into different regions of the Tiantan strain respectively, such as HA region, to ensure that gag can induce stronger cellular immunity.

### Example 6: Multivalent HIV vaccine that expresses different antigens in several recombination regions

### 1. Construction of transfer plasmid pVI76-gag

Using the genomic DNA of vaccinia virus Tiantan strain VTT (the virus was provided by National Vaccine and Serum Institute of Beijing) as the template, primer A55R-for with EcoRI restriction site (5' CATACGCGATCAGAATTCATCGTTGACATCTAGTATTGA TAG 3', SEQ ID NO:16) and primer A55R-Rev with StuI, XhoI and AscI restriction sites (5' TAAGGCCTCTCGAGGCGCGCCCTATCAACTACCTATAAAACTTTCC 3', SEQ ID NO:17) were used to amplify A55R fragment by PCR. The amplification conditions were as follows: 95°C 5 min; 95°C 30s, 55°C 30s, 72°C 1 min, 30 cycles; 72°C 5 min. The size of A55R fragment was 621 bp. Primer A57R-for with XhoII and PacI restriction sites (5' GAGAACCTCGAGTTAATTAATGACTTACATAAATGTCTGGGATAG 3', SEQ ID NO:18) and primer A57R-Rev with StuI restriction site (5' TCTAGGCCTTGTTAAAATACATTCTAATACGGTC 3', SEQ ID NO:19) were used to amplify A57R fragment by PCR. The amplification conditions were as follows: 95°C 5 min; 95°C 30s, 55°C 30s, 72°C 1 min, 30 cycles; 72°C 5 min. The size of A57R fragment was 600 bp. Primer A55R-DR-for with XhoII restriction site (5' AAATCTCGAGAGAATTAATCCCGCTCTATGGTCAG 3', SEQ ID NO:20) and primer A55R-DR-Rev with HindI restriction site (5' GCGAAGCTTTTGTTCTATCAACTACCTATAAAAC 3', SEQ ID NO:21) were used to amplify A55R-DR fragment by PCR. The amplification conditions were as follows: 95°C 5 min; 95°C 30s, 55°C 30s, 72°C 30s, 30 cycles; 72°C 2 min. The size of A55R-DR fragment was 279 bp.

The A55R fragment obtained by the PCR amplification was double digested by *Xho*I and *EcoR*I*.* The A57R fragment was double digested by *Xho*I and *Stu*I*.* The digested fragments were then ligated into the plasmid pUC57 (Genscript, Cat. SD1176), which was double digested by *Xho*I and *Stu*I*.* A pUC57-A55RA57R plasmid was obtained. The A55R and A57R fragments were sequenced, and the results were showed in SEQ ID NO:5 and SEQ ID NO:6, respectively.

The plasmid pLW73-Neo-I8RDR (plasmid sequence is shown in SEQ ID NO:7) was double digested by *Asc*I and *Xho*I*,* and a fragment Neo-GFP was obtained. The fragment Neo-GFP was ligated into the plasmid pUC57-A55RA57R, which was digested by the same restriction enzymes. A transfer plasmid pUC57-A55RA57R-GN was obtained. The size of the plasmid was 5763bp.

PCR amplified fragment A55R-DR was double digested by enzymes *Hind*III and *Xho*I*,* and was then cloned into a plasmid pSC65, which was digested by the same restriction enzymes. A recombinant plasmid pSC65-A55R-DR was obtained. The A55R-DR fragment was sequenced, and the result was shown in SEQ ID NO:8. The plasmid pSC65-A55R-DR was double digested by *Pac*I and *Xho*I*,* and a fragment pE/L-DR was obtained. The fragment was ligated into the plasmid pUC57-A55RA57R-GN, which was double digested by *Pac*I and *Xho*I*.* A plasmid pVI76 was obtained. The plasmid size was 6117bp. The plasmid was sequenced, and the result was shown in SEQ ID NO:9.

The Gag fragment (SEQ ID NO:2) and the plasmid pVI76 were double digested by KpnI and PacI and then ligated. A transfer plasmid pVI76-gag was obtained. KpnI and PacI restriction enzymes were used for double digestion verification. The result showed that, the sizes of the enzyme digestion products were 1488bp and 6117bp respectively, indicating that Gag gene was correctly inserted into plasmid pVI76.

### 2. Construction and purification of recombinant viruses

The recombinant virus TT-EnvTK (CGMCC No. 9808) was inoculated at a viral exposure amount of MOI=1 to infect chicken embryo fibroblasts (CEFs) of 80-90% confluency, which were then cultivated at 37°C, 5% CO₂ for 2 hours. Liposome was used to transfect the recombinant plasmid pVI76-gag into the cells, according to the process delineated in the instruction manual of the kit (Invitrogen, Lipofectamine® 2000 transfection Reagent, Cat. 11668-027). The recombinant plasmid underwent homologous recombination with TT-EnvTK in HA region in the cell. 24 hours after the transfection, the cell sample was collected and frozen-thawed for 3 times.

The selection process for recombinant viruses was the same as Example 2, the purified recombinant vaccinia virus TT-TK+/EG was obtained through 3 generations of green fluorescence plaques and 5 generations of white plaques selection purification.

### 3. PCR identification of recombinant viruses

The genomic DNA of the recombinant virus TT-TK+/EG was extracted, and identification primers tkL-up (SEQ ID NO:14) and tkR-low (SEQ ID NO:15), SQEA57R-rev (5' TGTTAAAATACATTCTAATACGGTC 3', SEQ ID NO:22) and SQE55R-for (5' ATCGTTGACATCTAGTATTGATAG 3', SEQ ID NO:23) were used for PCR identification. The results showed that, the sizes of the fragments amplified by the two pairs of primers were 4966bp and 3047bp respectively, indicating that gp145 2M and HSV-TK were correctly recombined into the TK region of VTT genome, and Gag gene was correctly recombined into the HA region of VTT genome.

### 4. The expression of the target gene in the recombinant vaccinia virus

The 1-5# clones of the recombinant vaccinia virus TT-TK+/EG were respectively inoculated at a dose of 1PFU/cell to infect CEF cells, and the cells were collected after being cultivated for 24 hours. 1ml of protein extraction buffer (1% SDS, 1mmol/L PMSF, 20mmol/L Tris-Cl pH7.0, 1% β-mercaptoethanol) was added to the cells, and immediately blended, and repeatedly frozen-thawed for 3 times. PAGE electrophoresis and membrane transfer were performed, and 5% skim milk was used for blocking at the room temperature for 2 hours. HIV-1 SF2 GP160 anti-serum and HIV-1 Gag anti-serum (both from NIH AIDS Research & Reference Reagent Program) were used to respectively detect the expressions of HIV-1 Gp145 2M and Gag. A primary antibody diluted by 5% skim milk was incubated at the room temperature for 2 hours. PBS membrane was washed for 3 times, and each time for 10 min. A corresponding secondary antibody labeled by horseradish peroxidase was added, diluted at 1:5000 using 5% skim milk, and incubated at the room temperature for 1 hour. PBS membrane was washed for 3 times, and each time for 10 min. After ECL color development, the results showed that, 1-5# recombinant viruses can all be detected of the HIV-1Gp145 2M band of about 140 kD and the HIV-1 Gag band of about 55 kD, indicating that the recombinant viruses can correctly express the Gp145 2M and Gag genes.

### References

1. Redfield R R, Wright D C, James W D, et al., Disseminated vaccinia in a military recruit with human immunodeficiency virus (HIV) disease. N Engl J Med, 1987. 316(11): 673-676.
2. Kesson A M, Ferguson J K, Rawlinson W D, et al., Progressive vaccinia treated with ribavirin and vaccinia immune globulin. Clin Infect Dis, 1997. 25(4): 911-914.
3. Austin E A and Huber B E, A first step in the development of gene therapy for colorectal carcinoma: cloning, sequencing, and expression of Escherichia coli cytosine deaminase. Mol Pharmacol, 1993. 43(3): 380-387.
4. Bridgewater J A, Springer C J, Knox R J, et al., Expression of the bacterial nitroreductase enzyme in mammalian cells renders them selectively sensitive to killing by the prodrug CB1954. Eur J Cancer, 1995. 31A(13-14): 2362-2370.
5. Patterson A V, Zhang H, Moghaddam A, et al., Increased sensitivity to the prodrug 5'-deoxy-5-fluorouridine and modulation of 5-fluoro-2'-deoxyuridine sensitivity in MCF-7 cells transfected with thymidine phosphorylase. Br J Cancer, 1995. 72(3): 669-675.
6. Parker W B, King S A, Allan P W, et al., In vivo gene therapy of cancer with E. coli purine nucleoside phosphorylase. Hum Gene Ther, 1997. 8(14): 1637-1644.
7. Kraiselburd E, Thymidine kinase gene transfer by herpes simplex virus. Bull Cancer, 1976. 63(3): 393-398.
8. Reardon J E, Herpes simplex virus type 1 and human DNA polymerase interactions with 2'-deoxyguanosine 5'-triphosphate analogues. Kinetics of incorporation into DNA and induction of inhibition. JBiol Chem, 1989. 264(32): 19039-19044.
9. Matthews T and Boehme R, Antiviral activity and mechanism of action of ganciclovir. Rev Infect Dis, 1988. 10 Suppl 3: S490-494.
10. Kern E R, In vitro activity of potential anti-poxvirus agents. Antiviral Res, 2003. 57(1-2):35-40.

## Claims

1. A recombinant replicative vaccinia virus, comprising, in its TK region, a first polynucleotide encoding an HSV-TK and a second polynucleotide encoding an HIV antigen.

2. The recombinant replicative vaccinia virus of claim 1, wherein the replicative vaccinia virus is a vaccinia virus Tiantan strain.

3. The recombinant replicative vaccinia virus of claim 1 or 2, wherein the first polynucleotide comprises a nucleotide sequence set forth in SEQ ID NO:3.

4. The recombinant replicative vaccinia virus of claim 1 or 2, wherein the first polynucleotide comprises a nucleotide sequence which is at least 80%, at least 85%, at least 90%, at least 95%, or at least 99% identical to the nucleotide sequence set forth in SEQ ID NO:3, and wherein the first polynucleotide encodes a TK from HSV-1.

5. The recombinant replicative vaccinia virus of any one of claims 1-4, wherein the second polynucleotide encodes a gp145 2M derived from 97CN001 which is the predominant HIV epidemic strain in China, and wherein the second polynucleotide comprises a nucleotide sequence set forth in SEQ ID NO:1.

6. The recombinant replicative vaccinia virus of any one of claims 1-4, wherein the second polynucleotide encodes a gp145 2M derived from 97CN001 which is the predominant HIV epidemic strain in China, or an immunogenic fragment thereof.

7. The recombinant replicative vaccinia virus of any one of claims 1-4, wherein the second polynucleotide comprises a nucleotide sequence which is at least 80%, at least 85%, at least 90%, at least 95%, or at least 99% identical to a nucleotide sequence as set forth in SEQ ID NO:1, and wherein the second polynucleotide encodes a gp145 2M from HIV-1.

8. The recombinant replicative vaccinia virus of any one of claims 1-7, further comprising a third polynucleotide encoding an additional HIV antigen.

9. The recombinant replicative vaccinia virus of claim 8, wherein the third polynucleotide is inserted into the HA region.

10. The recombinant replicative vaccinia virus of claim 9, wherein the third polynucleotide encodes a gag derived from 97CN001 which is the predominant HIV epidemic strain in China, and wherein the third polynucleotide comprises a nucleotide sequence set forth in SEQ ID NO:2.

11. The recombinant replicative vaccinia virus of claim 9, wherein the third polynucleotide encodes a gag derived from 97CN001 which is the predominant HIV epidemic strain in China, or an immunogenic fragment thereof.

12. The recombinant replicative vaccinia virus of claim 9, wherein the third polynucleotide comprises a nucleotide sequence which is at least 80%, at least 85%, at least 90%, at least 95%, or at least 99% identical to a nucleotide sequence set forth in SEQ ID NO:2, and wherein the third polynucleotide encodes a gag from HIV-1.

13. A recombinant replicative vaccinia virus, which is a vaccinia virus Tiantan strain which comprises a gene encoding a gp145 2M derived from 97CN001 which is the predominant HIV epidemic strain in China, and a gene encoding an HSV-TK, both genes being inserted into the TK region of the vaccinia virus Tiantan strain, and further comprises a gene encoding a gag derived from the 97CN001 inserted into the HA region of the vaccinia virus Tiantan strain.

14. A composition of a live vector HIV vaccine, comprising a recombinant replicative vaccinia virus of any one of claims 1-13 and a pharmaceutically acceptable carrier and/or adjuvant.

15. Use of a recombinant replicative vaccinia virus of any one of claims 1-13 in the preparation of a live vector HIV vaccine for the prevention and/or treatment of HIV infection in a subject.

16. The use of claim 15, wherein said subject is an immunocompromised patient.

17. The use of claim 16, wherein said subject has congenital immunodeficiency, has HIV infection, or is undergoing immunosuppressive therapy.

18. A kit comprising a plurality of components and an instruction indicating an immunization procedure, wherein one of the components is the composition of live vector HIV vaccine of claim 14.


SEQUENCE LISTING

<110>  NATIONAL CENTER FOR AIDS/STD CONTROL AND PREVENTION, CHINESE CENTER
FOR DISEASE CONTROL AND PREVENTION

<120>  REPLICATIVE VACCINIA VIRAL VIRUS VECTOR HIV VACCINE

<130>  P6067942PCT/EP

<140>  EP15852404.1
<141>  2015-10-19

<150>  CN 201410558649.5
<151>  2014-10-20

<160>  23

<170>  PatentIn version 3.3

<210>  1
<211>  2097
<212>  DNA
<213>  HIV-1

<400>  1
atggacaggg ccaagctgct gctgctgctg ctgctgctgc tgctgcccca ggcccaggcc      60

gtgggcaacc tgtgggtgac cgtgtactac ggcgtgcccg tgtggaaggg cgccaccacc     120

accgagttct gcgccagcga cgccaaggcc tacgacaccg aggtgcacaa cgtgtgggcc     180

acccacgcct gcgtgcccgc cgaccccaac ccccaggaga tggtgctgga gaacgtgacc     240

gagaacttca acatgtggaa gaacgagatg gtgaaccaga tgcaggagga cgtcatcagc     300

ctgtgggacc agagcctgaa gccctgcgtg aagctgaccc ccctgtgcgt gaccctggag     360

tgcaggaacg tgagcagcaa ccaggacacc taccacgaga cctaccacga gagcatgaag     420

gagatgaaga actgcagctt caacgccacc accgtggtga gggacaggaa gcagaccgtg     480

tacgccctgt tctacaggct ggacatcgtg cccctgacca agaagaacta cagcgagaac     540

agcagcgagt actacaggct gatcaactgc aacaccagcg ccatcaccca ggcctgcccc     600

aaggtgacct tcgaccccat ccccatccac tactgcaccc ccgccggcta cgccatcctg     660

aagtgcaacg acaagatctt caacggcacc ggcccctgcc acaacgtgag caccgtgcag     720

tgcacccacg gcatcaagcc cgtggtgagc acccagctgc tgctgaacgg cagcctggcc     780

gagggcgaga tcatcatcag gagcgagaac ctgaccaaca acgtgaaaac catcatcgtg     840

cacctgaacc agagcgtgga gatcgtgtgc accaggcccg gcaacaacac caggaagagc     900

atcaggatcg gccccggcca gaccttctac gccaccggcg acatcatcgg cgacatcagg     960

caggcccact gcaacatcag cgaggacaag tggaacgaga ccctgcagag ggtgagcaag    1020

aagcttgccg agcacttcca gaacaagacc atcaagttcg ccagcagcag cggcggcgac    1080

ctggaggtga ccacccacag cttcaactgc aggggcgagt tcttctactg caacaccagc    1140

ggcctgttca acggcgccta cacccccaac ggcaccaaga gcaacagcag cagcatcatc 1200

accatcccct gcaggatcaa gcagatcatc aacatgtggc aggaggtggg cagggccatg 1260

tacgcccctc ccatcaaggg caacatcacc tgcaagagca acatcaccgg cctgctgctg 1320

gtgagggacg gcggcaccga gcccaacgac accgagacct tcaggcccgg cggcggcgac 1380

atgaggaaca actggaggag cgagctgtac aagtacaagg tggtggagat caagcccctg 1440

ggcgtggccc ccaccaccac caagaggagg gtggtggaga gggagaagag ggccgtgggc 1500

atcggcgccg tgttcctggg cttcctgggc gtggccggca gcaccatggg cgccgccagc 1560

atcaccctga ccgtgcaggc caggcagctg ctgagcggca tcgtgcagca gcagagcaac 1620

ctgctgaggg ccatcgaggc ccagcagcac ctgctgcagc tgaccgtgtg gggcatcaag 1680

cagctgcaga ccagggtgct ggccatcgag aggtacctga aggaccagca gctgctgggc 1740

atctggggct gcagcggcaa gctgatctgc accaccgccg tgccctggaa cagcagctgg 1800

agcaacaaga gccagaagga gatctgggac aacatgacct ggatgcagtg ggacaaggag 1860

atcagcaact acaccaacac cgtgtacagg ctgctggagg agagccagaa ccagcaggag 1920

aggaacgaga aggacctgct ggccctggac agctggaaga acctgtggag ctggttcgac 1980

atcaccaact ggctgtggta catcaagatc ttcatcatca tcgtgggcgg cctgatcggc 2040

ctgaggatca tcttcgccgt gctgagcatc gtgaacaggg tgaggcaggg ctactga 2097

<210> 2
<211> 1488
<212> DNA
<213> HIV-1

<400> 2
atgggtgcga gagcgtcaat attaagaggg ggaaaattag ataaatggga aaaaattagg 60

ttaaggccag ggggaaagaa acactatatg ctaaaacacc tagtatgggc aagctgggag 120

ctggaaagat ttgcacttaa ccctggcctt ttagagacat cagaaggctg taaacaaata 180

atgaaacagc tacaaccagc tcttcagaca ggaacagagg aacttagatc attattcaac 240

acagtagcaa ctctctattg tgtacataca gagatagatg tacgagacac caaagaagcc 300

ttagacaaga tagaggaaga acaaaacaaa attcagcaaa aaacacagca ggcaaaggag 360

gctgacggga aggtcagtca aaattatcct atagtacaga atctccaagg gcaaatggta 420

catcagccca tatcacctag aactttaaat gcatgggtaa aagcggtaga agagaaggct 480

tttagcccag aagtaatacc catgttttca gcgttatcag aaggagccac cccacaagat 540

ttaaacacca tgctaaacac agtgggggga catcaagcag ccatgcaaat attaaaagat 600

accatcaatg aagaggctgc agaatgggat agattacatc cagtacatgc agggcctatt 660

gcaccaggcc aaatgagaga accaagggga agtgacatag caggaactac tagtaaccta 720

caggaacaaa tagcatggat gacgagtaac ccacctgttc cagtaggaga catctataaa 780

agatggataa ttctgggatt aaataaaata gtaagaatgt atagccctac cagcattctg 840

gacataaaac aagggccaaa ggaacccttt agagactatg tagaccggtt ctttaaaact 900

ttaagagcgg aacaagctac acaagatgta aaaaattgga tgacagacac cttgttggtc 960

caaaatgcga acccagattg taagaccatt ttaagagcat taggaccagg ggcttcaata 1020

gaagaaatga tgacagcatg tcagggagtg ggaggaccta gccataaagc aaaagtgttg 1080

gccgaggcaa tgagccaaac aaacagtgcc atactgatgc agagaagcaa ttttaaaggc 1140

tctaaaagaa ttgttaaatg tttcaactgt ggcaagggag ggcacatagc cagaaattgc 1200

agggccccta ggaaaaaggg ctgttggaaa tgtggaaaag aaggacacca aatgaaagat 1260

tgtactgaga gacaggccaa ttttttaggg aaaatctggc cctcccacaa gggaaggcca 1320

gggaattttc ttcagaacag accagagcca acagccccac cagaggagag cttcaggttt 1380

ggggaagaga caacaactcc atctcagaag caggagccaa tagacaagga actatatcct 1440

ttaacttccc tcaaatcact ctttggcaac gacccctcgt cacaataa 1488

<210> 3
<211> 1154
<212> DNA
<213> HSV-1

<400> 3
atggcttcgt acccctgcca tcagcacgcg tctgcgttcg accaggctgc gcgttctcgc 60

ggcaatagca accgacgtac ggcgttgcgc cctcgccggc agcaagaagc cacggaagtc 120

cgcccggagc agataatgcc cacgctactg cgggtttata tagacggtcc ccacgggatg 180

gggaaaacca ccaccacgca actgctggtg gccctgggtt cgcgcgacga tatcgtctac 240

gtacccgagc cgatgactta ctggcgggtg ctgggggctt ccgagacaat cgcgaacatc 300

tacaccacac aacaccgcct cgaccagggt gagatatcgg ccggggacgc ggcggtggta 360

atgacaagcg cccagataac aatgggcatg ccttatgccg tgaccgacgc cgttctggct 420

cctcatatcg ggggggaggc tgggagctca catgccccgc ccccggccct caccctcatc 480

ttcgaccgcc atcccatcgc cgccctcctg tgctacccgg ccgcgcgata ccttatgggc 540

agcatgaccc cccaggccgt gctggcgttc gtggccctca tcccgccgac cttgcccggc 600

acaaacatcg tgttgggggc ccttccggag gacagacaca tcgaccgcct ggccaaacgc 660

cagcgccccg gcgagcggct tgacctggct atgctggccg cgattcgccg cgtttacggg 720

ctgcttgcca atacggtgcg gtatctgcag ggcggcgggt cgtggcggga ggattgggga 780

cagctttcgg ggacggccgt gccgccccag ggtgccgagc cccagagcaa cgcgggccca 840

cgaccccata tcggggacac gttatttacc ctgtttcggg cccccgagtt gctggccccc 900

aacggcgacc tgtacaacgt gtttgcctgg gccttggacg tcttggccaa acgcctccgt 960

cccatgcacg tctttatcct ggattacgac caatcgcccg ccggctgccg ggacgccctg 1020

ctgcaactta cctccgggat gatccagacc cacgtcacca ccccaggctc cataccgacg 1080

atctgcgacc tggcgcgcac gtttgcccgg gagatggggg aggctaacca ccaccaccac 1140

caccactgaa agct 1154

<210> 4
<211> 6450
<212> DNA
<213> Artificial

<220>
<223> pSC65-DR-GN

<400> 4
agcttttggg atcaataaat ggatcacaac cagtatctct taacgatgtt cttcgcagat 60

gatgattcat tttttaagta tttggctagt caagatgatg aatcttcatt atctgatata 120

ttgcaaatca ctcaatatct agactttctg ttattattat tgatccaatc aaaaaataaa 180

ttagaagccg tgggtcattg ttatgaatct ctttcagagg aatacagaca attgtcaaaa 240

ttcacagact ttcaagattt taaaaaactg tttaacaagg tccctattgt tacagatgga 300

agggtcaaac ttaataaagg atatttgttc gactttgtga ttagtttgat gcgattcaaa 360

aaagaatcct ctctagctac caccgcaata gatcctgtta gatacataga tcctcgtcgc 420

aatatcgcat tttctaacgt gatggatata ttaaagtcga ataaagtgaa caataattaa 480

ttctttattg tcatcatgaa cggcggacat attcagttga taatcggccc catgttttca 540

ggtaaaagta cagaattaat tagacgagtt agacgttatc aaatagctca atataaatgc 600

gtgactataa aatattctaa cgataatagt tacggaacgg gactatggac gcatgataag 660

aataattttg aagcattgga agcaactaaa ctatgtgatc tcttggaatc aattacagat 720

ttctccgtga taggtatcga tgaaggacag ttctttccag acattgttga attagatcga 780

taaaaattaa ttaattaccc gggtaccagg cctagtactg tcgacttcga gcttatttat 840

attccaaaaa aaaaaaataa aatttcaatt tttaagcttt acatcattcc aaacccaccc 900

gctttttata gtaagttttt cacccataaa taataaatac aataattaat ttctcgtaaa 960

agtagaaaat atattctaat ttattgcacg gtaaggaagt agatcataac tcgagtctgt 1020

gagcgtatgg caaacgaagg aaaaatagtt atagtagccg cactcgatgg gacatttcaa 1080

cgtaaaccgt ttaataatat tttgaatctt attccattat ctgaaatggt ggtaaaacta 1140

actgctgtgt gtatgaaatg ctttaaggag gcttcctttt ctaaacgatt gggtgaggaa 1200

accgagatag aaataatagg aggtaatgat atgtatcaat cggtgtgtaa aaagtgatac 1260

atcgactcat aatgcctttc attttgtttt tttctatgct ataaatggtg agcaagggcg 1320

aggagctgtt caccggggtg gtgcccatcc tggtcgagct ggacggcgac gtaaacggcc 1380

acaagttcag cgtgtccggc gagggcgagg gcgatgccac ctacggcaag ctgaccctga 1440

agttcatctg caccaccggc aagctgcccg tgccctggcc caccctcgtg accaccctga 1500

cctacggcgt gcagtgcttc agccgctacc ccgaccacat gaagcagcac gacttcttca 1560

agtccgccat gcccgaaggc tacgtccagg agcgcaccat cttcttcaag gacgacggca 1620

actacaagac ccgcgccgag gtgaagttcg agggcgacac cctggtgaac cgcatcgagc 1680

tgaagggcat cgacttcaag gaggacggca acatcctggg gcacaagctg gagtacaact 1740

acaacagcca caacgtctat atcatggccg acaagcagaa gaacggcatc aaggtgaact 1800

tcaagatccg ccacaacatc gaggacggca gcgtgcagct tgccgaccac taccagcaga 1860

acacccccat cggcgacggc cccgtgctgc tgcccgacaa ccactacctg agcacccagt 1920

ccgccctgag caaagacccc aacgagaagc gcgatcacat ggtcctgctg gagttcgtga 1980

ccgccgccgg gatcactctc ggcatgcacg agctgtacaa gtaagagcga attcggtctg 2040

gcgagaccat tacccacatg ataagagatt gtatcagttt cgtagtcttg agtattggta 2100

ttactatata gtatatagac gtctagagga tcgatcccca gattacaaac aactaggaaa 2160

ttggtttata atgtataatt tttttagttt ttatagattc tttattctat acttaaaaaa 2220

tgaaaataaa tacaaaggtt cttgagggtt gtgttaaatt gaaagcgaga aataatcata 2280

aattatttca ttatcgcgat atccgttaag tttgtatcgt aatagatcgg gataattcct 2340

gcagccaata tgggatcggc cattgaacaa gatggattgc acgcaggttc tccggccgct 2400

tgggtggaga ggctattcgg ctatgactgg gcacaacaga caatcggctg ctctgatgcc 2460

gccgtgttcc ggctgtcagc gcaggggcgc ccggttcttt ttgtcaagac cgacctgtcc 2520

ggtgccctga atgaactgca ggacgaggca gcgcggctat cgtggctggc cacgacgggc 2580

gttccttgcg cagctgtgct cgacgttgtc actgaagcgg gaagggactg gctgctattg 2640

ggcgaagtgc cggggcagga tctcctgtca tctcaccttg ctcctgccga gaaagtatcc 2700

atcatggctg atgcaatgcg gcggctgcat acgcttgatc cggctacctg cccattcgac 2760

caccaagcga aacatcgcat cgagcgagca cgtactcgga tggaagccgg tcttgtcgat 2820

caggatgatc tggacgaaga gcatcagggg ctcgcgccag ccgaactgtt cgccaggctc 2880

aaggcgcgca tgcccgacgg cgatgatctc gtcgtgaccc atggcgatgc ctgcttgccg 2940

aatatcatgg tggaaaatgg ccgcttttct ggattcatcg actgtggccg gctgggtgtg 3000

gcggaccgct atcaggacat agcgttggct acccgtgata ttgctgaaga gcttggcggc 3060

gaatgggctg accgcttcct cgtgctttac ggtatcgccg ctcccgattc gcagcgcatc 3120

gccttctatc gccttcttga cgagttcttc tgaggggatc aattctctag agctcgctga 3180

tcagcctcga ctgtgccttc tagttgccag ccatctgttg tttgcccctc ccccgtgcct 3240
tccttgaccc tggaaggtgc cactcccact gtcctttcct aataaaatga cggatccttc 3300
tgtgagcgta tggcaaacga aggaaaaata gttatagtag ccgcactcga tgggacattt 3360
caacgtaaac cgtttaataa tattttgaat cttattccat tatctgaaat ggtggtaaaa 3420
ctaactgctg tgtgtatgaa atgctttaag gaggcttcct tttctaaacg attgggtgag 3480
gaaaccgaga tagaaataat aggaggtaat gatatgtatc aatcggtgtg tagaaagtgt 3540
tacatcgact cataatatta tattttttat ctaaaaaact aaaaataaac attgattaaa 3600
ttttaatata atacttaaaa atggatgttg tgtcgttaga taaaccgttt atgtattttg 3660
aggaaattga taatgagtta gattacgaac cagaaagtgc aaatgaggtc gcaaaaaaac 3720
tgccgtatca aggacagtta aaactattac taggagaatt atttttcttt agtaagttac 3780
agcgacacgg tatattagat ggtgccaccg tagtgtatat aggatctgct cccggtacac 3840
atatacgtta tttgagagat catttctata atttaggagt gatcatcaaa tggatgctaa 3900
ttgacggccg ccatcatgat cctattttaa atggattgcg tgatgtgact ctagtgactc 3960
ggttcgttga tgaggaatat ctacgatcca tcaaaaaaca actgcatcct tctaagatta 4020
ttttaatttc tgatgtgaga tccaaacgag gaggaaatga acctagtacg gcggatttac 4080
taagtaatta cgctctacta aatgtcatga ttagtatttt aaaccccgtg gcgtctagtc 4140
ttaaatggag atgcccgttt ccagatcaat ggatcaagga cttttatatc ccacacggta 4200
ataaaatgtt acaacctttt gctccttcat attcagctga aatgagatta ttaagtattt 4260
ataccggtga gaacatgaga ctgactcggg ccgcgttgct ggcgtttttc cataggctcc 4320
gcccccctga cgagcatcac aaaaatcgac gctcaagtca gaggtggcga aacccgacag 4380
gactataaag ataccaggcg tttccccctg gaagctccct cgtgcgctct cctgttccga 4440
ccctgccgct taccggatac ctgtccgcct ttctcccttc gggaagcgtg gcgctttctc 4500
aatgctcacg ctgtaggtat ctcagttcgg tgtaggtcgt tcgctccaag ctgggctgtg 4560
tgcacgaacc ccccgttcag cccgaccgct gcgccttatc cggtaactat cgtcttgagt 4620
ccaacccggt aagacacgac ttatcgccac tggcagcagc cactggtaac aggattagca 4680
gagcgaggta tgtaggcggt gctacagagt tcttgaagtg gtggcctaac tacggctaca 4740
ctagaaggac agtatttggt atctgcgctc tgctgaagcc agttaccttc ggaaaaagag 4800
ttggtagctc ttgatccggc aaacaaacca ccgctggtag cggtggtttt tttgtttgca 4860
agcagcagat tacgcgcaga aaaaaaggat ctcaagaaga tcctttgatc ttttctacgg 4920
ggtctgacgc tcagtggaac gaaaactcac gttaagggat tttggtcatg agattatcaa 4980
aaaggatctt cacctagatc cttttaaatt aaaaatgaag ttttaaatca atctaaagta 5040
tatatgagta aacttggtct gacagttacc aatgcttaat cagtgaggca cctatctcag 5100

cgatctgtct atttcgttca tccatagttg cctgactccc cgtcgtgtag ataactacga 5160

tacgggaggg cttaccatct ggccccagtg ctgcaatgat accgcgagac ccacgctcac 5220

cggctccaga tttatcagca ataaaccagc cagccggaag ggccgagcgc agaagtggtc 5280

ctgcaacttt atccgcctcc atccagtcta ttaattgttg ccgggaagct agagtaagta 5340

gttcgccagt taatagtttg cgcaacgttg ttgccattgc tgcaggcatc gtggtgtcac 5400

gctcgtcgtt tggtatggct tcattcagct ccggttccca acgatcaagg cgagttacat 5460

gatcccccat gttgtgcaaa aaagcggtta gctccttcgg tcctccgatc gttgtcagaa 5520

gtaagttggc cgcagtgtta tcactcatgg ttatggcagc actgcataat tctcttactg 5580

tcatgccatc cgtaagatgc ttttctgtga ctggtgagta ctcaaccaag tcattctgag 5640

aatagtgtat gcggcgaccg agttgctctt gcccggcgtc aacacgggat aataccgcgc 5700

cacatagcag aactttaaaa gtgctcatca ttggaaaacg ttcttcgggg cgaaaactct 5760

caaggatctt accgctgttg agatccagtt cgatgtaacc cactcgtgca cccaactgat 5820

cttcagcatc ttttactttc accagcgttt ctgggtgagc aaaaacagga aggcaaaatg 5880

ccgcaaaaaa gggaataagg gcgacacgga aatgttgaat actcatactc ttcctttttc 5940

aatattattg aagcatttat cagggttatt gtctcatgag cggatacata tttgaatgta 6000

tttagaaaaa taaacaaata ggggttccgc gcacatttcc ccgaaaagtg ccacctgacg 6060

tctaagaaac cattattatc atgacattaa cctataaaaa taggcgtatc acgaggccct 6120

ttcgtcttcg aataaatacc tgtgacggaa gatcacttcg cagaataaat aaatcctggt 6180

gtccctgttg ataccgggaa gccctgggcc aacttttggc gaaaatgaga cgttgatcgg 6240

cacgtaagag gttccaactt tcaccataat gaaataagat cactaccggg cgtatttttt 6300

gagttatcga gattttcagg agctaaggaa gctaaaatgg agaaaaaaat cactggatat 6360

accaccgttg atatatccca atggcatcgt aaagaacatt ttgaggcatt tcagtcagtt 6420

gctcaatgta cctataacca gaccgttcag 6450

<210> 5
<211> 622
<212> DNA
<213> Vaccinia virus

<400> 5
atcgttgaca tctagtattg atagatggaa gccatcaaaa ccatattggc agaagtatgc 60

taaaatgcgc gaaccaaaat gtgatatggg ggttgcgatg ttaaacggat taatatatgt 120

catgggtgga atcgttaaag gtgacacgtg taccgacgca ctagagagtt tatcagaaga 180

tggatggatg aagcatcaac gtcttccaat aaaaatgtcc aatatgtcga cgattgttca 240

tgatggcaag atttatatat ctggaggtta caacaatagt agtgtagtta atgtaatatc 300

gaatctagtc cttagctata atccgatata tgatgaatgg accaaattat catcattaaa 360

tattcctaga attaatcccg ctctatggtc agcgcataat aaattatatg taggaggagg 420

aatatctgat gatgttcgaa ctaatacatc tgagacatac gacaaagaaa aagattgttg 480

gacattggat aatggtcacg tgttaccacg caattatata atgtataaat gcgaaccgat 540

taaacataaa tatccattgg aaaaaacaca gtacacgaat gattttctaa agtatttgga 600

aagttttata ggtagttgat ag 622

<210> 6
<211> 587
<212> DNA
<213> Vaccinia virus

<400> 6
tgacttacat aaatgtctgg gatagtaaaa tctatcatat tgagcggacc atctggttca 60

ggaaagacag ccatagccaa aagactatgg gaatatattt ggatttgtgg tgtcccatac 120

cactagattt cctcgtccta tggaacgaga aggtgtcgat taccattacg ttaacagaga 180

ggccatctgg aagggaatag ccgccggaaa ctttctagaa catactgagt ttttaggaaa 240

tatttacgga acttctaaaa cagctgtgaa tacagcggct attaataatc gtatttgtgt 300

gatggatcta aacatcgacg gtgttagaag ttttaaaaat acttacctaa tgccttactc 360

ggtgtatata agacctacct ctcttaaaat ggttgagacc aagcttcgtt gtagaaacac 420

tgaagcggat gatgagattc atcgtcgtgt gatgttggca aaaactgaca tggatgaggc 480

aggtgaagcc ggtctattcg acactattat tattgaagat gatgtgaatt tagcatatag 540

taagttaatt cagatactac aggaccgtat tagaatgtat tttaaca 587

<210> 7
<211> 6188
<212> DNA
<213> Artificial

<220>
<223> pLW73-Neo-I8RDR

<400> 7
gaattccctg ggacatacgt atatttctat gatctgtctt atatgaagtc tatacagcga 60

atagattcag aatttctaca taattatata ttgtacgcta ataagtttaa tctaacactc 120

cccgaagatt tgtttataat ccctacaaat ttggatattc tatggcgtac aaaggaatat 180

atagactcgt tcgatattag tacagaaaca tggaataaat tattatccaa ttattatatg 240

aagatgatag agtatgctaa actttatgta ctaagtccta ttctcgctga ggagttggat 300

aattttgaga ggacgggaga attaactagt attgtacaag aagccatttt atctctaaat 360

ttacgaatta agattttaaa ttttaaacat aaagatgatg atacgtatat acacttttgt 420

aaaatattat tcggtgtcta taacggaaca aacgctacta tatattatca tagacctcta 480

acgggatata tgaatatgat ttcagatact atatttgttc ctgtagataa taactaaggc 540

gcgcctttca ttttgttttt ttctatgcta taaatggtga gcaagggcga ggagctgttc 600

accggggtgg tgcccatcct ggtcgagctg gacggcgacg taaacggcca caagttcagc 660

gtgtccggcg agggcgaggg cgatgccacc tacggcaagc tgaccctgaa gttcatctgc 720

accaccggca agctgcccgt gccctggccc accctcgtga ccaccctgac ctacggcgtg 780

cagtgcttca gccgctaccc cgaccacatg aagcagcacg acttcttcaa gtccgccatg 840

cccgaaggct acgtccagga gcgcaccatc ttcttcaagg acgacggcaa ctacaagacc 900

cgcgccgagg tgaagttcga gggcgacacc ctggtgaacc gcatcgagct gaagggcatc 960

gacttcaagg aggacggcaa catcctgggg cacaagctgg agtacaacta caacagccac 1020

aacgtctata tcatggccga caagcagaag aacggcatca aggtgaactt caagatccgc 1080

cacaacatcg aggacggcag cgtgcagctc gccgaccact accagcagaa cacccccatc 1140

ggcgacggcc ccgtgctgct gcccgacaac cactacctga gcacccagtc cgccctgagc 1200

aaagacccca acgagaagcg cgatcacatg gtcctgctgg agttcgtgac cgccgccggg 1260

atcactctcg gcatgcacga gctgtacaag taagagctct cccctcagaa gaactcgtca 1320

agaaggcgat agaaggcgat gcgctgcgaa tcgggagcgg cgataccgta aagcacgagg 1380

aagcggtcag cccattcgcc gccaagctct tcagcaatat cacgggtagc caacgctatg 1440

tcctgatagc ggtccgccac acccagccgg ccacagtcga tgaatccaga aaagcggcca 1500

ttttccacca tgatattcgg caagcaggca tcgccatggg tcacgacgag atcatcgccg 1560

tcgggcatgc gcgccttgag cctggcgaac agttcggctg gcgcgagccc ctgatgctct 1620

tcgtccagat catcctgatc gacaagaccg gcttccatcc gagtacgtgc tcgctcgatg 1680

cgatgtttcg cttggtggtc gaatgggcag gtagccggat caagcgtatg cagccgccgc 1740

attgcatcag ccatgatgga tactttctcg gcaggagcaa ggtgagatga caggagatcc 1800

tgccccggca cttcgcccaa tagcagccag tcccttcccg cttcagtgac aacgtcgagc 1860

acagctgcgc aaggaacgcc cgtcgtggcc agccacgata gccgcgctgc ctcgtcctgc 1920

agttcattca gggcaccgga caggtcggtc ttgacaaaaa gaaccgggcg cccctgcgct 1980

gacagccgga acacggcggc atcagagcag ccgattgtct gttgtgccca gtcatagccg 2040

aatagcctct ccacccaagc ggccggagaa cctgcgtgca atccatcttg ttcaatggcc 2100

gatcccatat tggctgcagg aattatcccg atctatacga tacaaactta acggatatcg 2160

cgataatgaa ataatttatg attatttctc gctttcaatt taacacaacc ctcaagaacc 2220

tttgtattta ttttcatttt ttaagtatag aataaagaat ctataaaaac taaaaaaatt 2280

atacattata aaccaatttc ctagttgttt gtaatctggg gatcgatcct ctagacatct 2340

atatactata tagtaatacc aatactcaag actacgaaac tgatacaatc tcttatcatg 2400

tgggtaatgg tctcgccaga ccaactggta agatatcgag ctcgaggacg ggagaattaa 2460

ctagtattgt acaagaagcc attttatctc taaatttacg aattaagatt ttaaatttta 2520

aacataaaga tgatgatacg tatatacact tttgtaaaat attattcggt gtctataacg 2580

gaacaaacgc tactatatat tatcatagac ctctaacggg atatatgaat atgatttcag 2640

atactatatt tgttcctgta gataataact aactcgaggc cgctggtacc caacctaaaa 2700

attgaaaata aatacaaagg ttcttgaggg ttgtgttaaa ttgaaagcga gaaataatca 2760

taaataagcc cggggatcct ctagagtcga cctgcagtca aactctaatg accacatctt 2820

tttttagaga tgaaaaattt tccacatctc cttttgtaga cacgactaaa cattttgcag 2880

aaaaaagttt attagtgttt agataatcgt atacttcatc agtgtagata gtaaatgtga 2940

acagataaaa ggtattcttg ctcaatagat tggtaaattc catagaatat attaatcctt 3000

tcttcttgag atcccacatc atttcaacca gagacgtttt atccaatgat ttacctcgta 3060

ctataccaca tacaaaacta gattttgcag tgacgtcgta tctggtattc ctaccaaaca 3120

aaattttact tttagttctt ttagaaaatt ctaaggtaga atctctattt gccaatatgt 3180

catctatgga attaccacta gcaaaaaatg atagaaatat atattgatac atcgcagctg 3240

gttttgatct actatacttt aaaaacgaat cagattccat aattgcctgt atatcatcag 3300

ctgaaaaact atgttttaca cgtattcctt cggcatttct ttttaatgat atatcttgtt 3360

tagacaatga taaagttatc atgtccatga gagacgcgtc tccgtatcgt ataaatattt 3420

cattagatgt tagacgcttc attaggggta tacttctata aggtttctta atcagtccat 3480

cattggttgc gtcaagaaca agcttgtctc cctatagtga gtcgtattag agcttggcgt 3540

aatcatggtc atagctgttt cctgtgtgaa attgttatcc gctcacaatt ccacacaaca 3600

tacgagccgg aagcataaag tgtaaagcct ggggtgccta atgagtgagc taactcacat 3660

taattgcgtt gcgctcactg cccgctttcg agtcgggaaa cctgtcgtgc cagctgcatt 3720

aatgaatcgg ccaacgcgcg gggagaggcg gtttgcgtat tgggcgctct tccgcttcct 3780

cgctcactga ctcgctgcgc tcggtcgttc ggctgcggcg agcggtatca gctcactcaa 3840

aggcggtaat acggttatcc acagaatcag gggataacgc aggaaagaac atgtgagcaa 3900

aaggccagca aaaggccagg aaccgtaaaa aggccgcgtt gctggcgttt ttcgataggc 3960

tccgcccccc tgacgagcat cacaaaaatc gacgctcaag tcagaggtgg cgaaacccga 4020

caggactata aagataccag gcgtttcccc ctggaagctc cctcgtgcgc tctcctgttc 4080

cgaccctgcc gcttaccgga tacctgtccg cctttctccc ttcgggaagc gtggcgcttt 4140

ctcatagctc acgctgtagg tatctcagtt cggtgtaggt cgttcgctcc aagctgggct 4200

gtgtgcacga accccccgtt cagcccgacc gctgcgcctt atccggtaac tatcgtcttg 4260

agtccaaccc ggtaagacac gacttatcgc cactggcagc agccactggt aacaggatta 4320

gcagagcgag gtatgtaggc ggtgctacag agttcttgaa gtggtggcct aactacggct 4380

acactagaag gacagtattt ggtatctgcg ctctgctgaa gccagttacc ttcggaaaaa 4440

gagttggtag ctcttgatcc ggcaaacaaa ccaccgctgg tagcggtggt ttttttgttt 4500

gcaagcagca gattacgcgc agaaaaaaag gatctcaaga agatcctttg atcttttcta 4560

cggggtctga cgctcagtgg aacgaaaact cacgttaagg gattttggtc atgagattat 4620

caaaaaggat cttcacctag atccttttaa attaaaaatg aagttttaaa tcaatctaaa 4680

gtatatatga gtaaacttgg tctgacagtt accaatgctt aatcagtgag gcacctatct 4740

cagcgatctg tctatttcgt tcatccatag ttgcctgact ccccgtcgtg tagataacta 4800

cgatacggga gggcttacca tctggcccca gtgctgcaat gataccgcga gacccacgct 4860

caccggctcc agatttatca gcaataaacc agccagccgg aagggccgag cgcagaagtg 4920

gtcctgcaac tttatccgcc tccatccagt ctattaattg ttgccgggaa gctagagtaa 4980

gtagttcgcc agttaatagt ttgcgcaacg ttgttggcat tgctacaggc atcgtggtgt 5040

cacgctcgtc gtttggtatg gcttcattca gctccggttc ccaacgatca aggcgagtta 5100

catgatcccc catgttgtgc aaaaaagcgg ttagctcctt cggtcctccg atcgttgtca 5160

gaagtaagtt ggccgcagtg ttatcactca tggttatggc agcactgcat aattctctta 5220

ctgtcatgcc atccgtaaga tgcttttctg tgactggtga gtactcaacc aagtcattct 5280

gagaatagtg tatgcggcga ccgagttgct cttgcccggc gtcaatacgg gataataccg 5340

cgccacatag cagaacttta aaagtgctca tcattggaaa acgttcttcg gggcgaaaac 5400

tctcaaggat cttaccgctg ttgagatcca gttcgatgta acccactcgt gcacccaact 5460

gatcttcagc atcttttact ttcaccagcg tttctgggtg agcaaaaaca ggaaggcaaa 5520

atgccgcaaa aaagggaata agggcgacac ggaaatgttg aatactcata ctcttccttt 5580

ttcaatatta ttgaagcatt tatcagggtt attgtctcat gagcggatac atatttgaat 5640

gtatttagaa aaataaacaa ataggggttc cgcgcacatt tccccgaaaa gtgccacctg 5700

acgtctaaga aaccattatt atcatgacat taacctataa aaataggcgt atcacgaggc 5760

cctttcgtct cgcgcgtttc ggtgatgacg gtgaaaacct ctgacacatg cagctcccgg 5820

agacggtcac agcttgtctg taagcggatg ccgggagcag acaagcccgt cagggcgcgt 5880

cagcgggtgt tggcgggtgt cggggctggc ttaactatgc ggcatcagag cagattgtac 5940

tgagagtgca ccatatgcgg tgtgaaatac cgcacagatg cgtaaggaga aaataccgca 6000

tcaggcgcca ttcgccattc aggctgcgca actgttggga agggcgatcg gtgcgggcct 6060

cttcgctatt acgccagctg gcgaaagggg gatgtgctgc aaggcgatta agttgggtaa 6120

cgccagggtt ttcccagtca cgacgttgta aaacgacggc cagtgaattg gatttaggtg 6180

acactata 6188

<210> 8
<211> 260
<212> DNA
<213> Vaccinia virus

<400> 8
agaattaatc ccgctctatg gtcagcgcat aataaattat atgtaggagg aggaatatct 60

gatgatgttc gaactaatac atctgagaca tacgacaaag aaaaagattg ttggacattg 120

gataatggtc acgtgttacc acgcaattat ataatgtata aatgcgaacc gattaaacat 180

aaatatccat tggaaaaaac acagtacacg aatgattttc taaagtattt ggaaagtttt 240

ataggtagtt gatagaacaa 260

<210> 9
<211> 6117
<212> DNA
<213> Artificial

<220>
<223> pVI76

<400> 9
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgaatt catcgttgac atctagtatt 420

gatagatgga agccatcaaa accatattgg cagaagtatg ctaaaatgcg cgaaccaaaa 480

tgtgatatgg gggttgcgat gttaaacgga ttaatatatg tcatgggtgg aatcgttaaa 540

ggtgacacgt gtaccgacgc actagagagt ttatcagaag atggatggat gaagcatcaa 600

cgtcttccaa taaaaatgtc caatatgtcg acgattgttc atgatggcaa gatttatata 660

tctggaggtt acaacaatag tagtgtagtt aatgtaatat cgaatctagt ccttagctat 720

aatccgatat atgatgaatg gaccaaatta tcatcattaa atattcctag aattaatccc 780

gctctatggt cagcgcataa taaattatat gtaggaggag gaatatctga tgatgttcga 840

actaatacat ctgagacata cgacaaagaa aaagattgtt ggacattgga taatggtcac 900

gtgttaccac gcaattatat aatgtataaa tgcgaaccga ttaaacataa atatccattg 960

gaaaaaacac agtacacgaa tgattttcta aagtatttgg aaagttttat aggtagttga 1020

tagggcgcgc ctttcatttt gtttttttct atgctataaa tggtgagcaa gggcgaggag 1080

ctgttcaccg gggtggtgcc catcctggtc gagctggacg gcgacgtaaa cggccacaag 1140

ttcagcgtgt ccggcgaggg cgagggcgat gccacctacg gcaagctgac cctgaagttc 1200

atctgcacca ccggcaagct gcccgtgccc tggcccaccc tcgtgaccac cctgacctac 1260

ggcgtgcagt gcttcagccg ctaccccgac cacatgaagc agcacgactt cttcaagtcc 1320

gccatgcccg aaggctacgt ccaggagcgc accatcttct tcaaggacga cggcaactac 1380

aagacccgcg ccgaggtgaa gttcgagggc gacaccctgg tgaaccgcat cgagctgaag 1440

ggcatcgact tcaaggagga cggcaacatc ctggggcaca agctggagta caactacaac 1500

agccacaacg tctatatcat ggccgacaag cagaagaacg gcatcaaggt gaacttcaag 1560

atccgccaca acatcgagga cggcagcgtg cagctcgccg accactacca gcagaacacc 1620

cccatcggcg acggccccgt gctgctgccc gacaaccact acctgagcac ccagtccgcc 1680

ctgagcaaag accccaacga gaagcgcgat cacatggtcc tgctggagtt cgtgaccgcc 1740

gccgggatca ctctcggcat gcacgagctg tacaagtaag agctctcccc tcagaagaac 1800

tcgtcaagaa ggcgatagaa ggcgatgcgc tgcgaatcgg gagcggcgat accgtaaagc 1860

acgaggaagc ggtcagccca ttcgccgcca agctcttcag caatatcacg ggtagccaac 1920

gctatgtcct gatagcggtc cgccacaccc agccggccac agtcgatgaa tccagaaaag 1980

cggccatttt ccaccatgat attcggcaag caggcatcgc catgggtcac gacgagatca 2040

tcgccgtcgg gcatgcgcgc cttgagcctg gcgaacagtt cggctggcgc gagcccctga 2100

tgctcttcgt ccagatcatc ctgatcgaca agaccggctt ccatccgagt acgtgctcgc 2160

tcgatgcgat gtttcgcttg gtggtcgaat gggcaggtag ccggatcaag cgtatgcagc 2220

cgccgcattg catcagccat gatggatact ttctcggcag gagcaaggtg agatgacagg 2280

agatcctgcc ccggcacttc gcccaatagc agccagtccc ttcccgcttc agtgacaacg 2340

tcgagcacag ctgcgcaagg aacgcccgtc gtggccagcc acgatagccg cgctgcctcg 2400

tcctgcagtt cattcagggc accggacagg tcggtcttga caaaaagaac cgggcgcccc 2460

tgcgctgaca gccggaacac ggcggcatca gagcagccga ttgtctgttg tgcccagtca 2520

tagccgaata gcctctccac ccaagcggcc ggagaacctg cgtgcaatcc atcttgttca 2580

atggccgatc ccatattggc tgcaggaatt atcccgatct atacgataca aacttaacgg 2640

atatcgcgat aatgaaataa tttatgatta tttctcgctt tcaatttaac acaaccctca 2700

agaacctttg tatttatttt cattttttaa gtatagaata aagaatctat aaaaactaaa 2760

aaaattatac attataaacc aatttcctag ttgtttgtaa tctggggatc gatcctctag 2820

acatctatat actatatagt aataccaata ctcaagacta cgaaactgat acaatctctt 2880

atcatgtggg taatggtctc gccagaccaa ctggtaagat atcgagctcg agagaattaa 2940

tcccgctcta tggtcagcgc ataataaatt atatgtagga ggaggaatat ctgatgatgt 3000

tcgaactaat acatctgaga catacgacaa agaaaaagat tgttggacat tggataatgg 3060

tcacgtgtta ccacgcaatt atataatgta taaatgcgaa ccgattaaac ataaatatcc 3120

attggaaaaa acacagtaca cgaatgattt tctaaagtat ttggaaagtt ttataggtag 3180

ttgatagaac aagtcgtccg caagcttaaa aattgaaatt ttattttttt tttttggaat 3240

ataaataagc tcgaagtcga cagtactagg cctggtaccc gggtaattaa ttaagactta 3300

cataaatgtc tgggatagta aaatctatca tattgagcgg accatctggt tcaggaaaga 3360

cagccatagc caaaagacta tgggaatata tttggatttg tggtgtccca taccactaga 3420

tttcctcgtc ctatggaacg agaaggtgtc gattaccatt acgttaacag agaggccatc 3480

tggaagggaa tagccgccgg aaactttcta gaacatactg agtttttagg aaatatttac 3540

ggaacttcta aaacagctgt gaatacagcg gctattaata atcgtatttg tgtgatggat 3600

ctaaacatcg acggtgttag aagttttaaa aatacttacc taatgcctta ctcggtgtat 3660

ataagaccta cctctcttaa aatggttgag accaagcttc gttgtagaaa cactgaagcg 3720

gatgatgaga ttcatcgtcg tgtgatgttg gcaaaaactg acatggatga ggcaggtgaa 3780

gccggtctat tcgacactat tattattgaa gatgatgtga atttagcata tagtaagtta 3840

attcagatac tacaggaccg tattaaggcc tgcatgcaag cttggcgtaa tcatggtcat 3900

agctgtttcc tgtgtgaaat tgttatccgc tcacaattcc acacaacata cgagccggaa 3960

gcataaagtg taaagcctgg ggtgcctaat gagtgagcta actcacatta attgcgttgc 4020

gctcactgcc cgctttccag tcgggaaacc tgtcgtgcca gctgcattaa tgaatcggcc 4080

aacgcgcggg gagaggcggt ttgcgtattg ggcgctcttc cgcttcctcg ctcactgact 4140

cgctgcgctc ggtcgttcgg ctgcggcgag cggtatcagc tcactcaaag gcggtaatac 4200

ggttatccac agaatcaggg gataacgcag gaaagaacat gtgagcaaaa ggccagcaaa 4260

aggccaggaa ccgtaaaaag gccgcgttgc tggcgttttt ccataggctc cgcccccctg 4320

acgagcatca caaaaatcga cgctcaagtc agaggtggcg aaacccgaca ggactataaa 4380

gataccaggc gtttccccct ggaagctccc tcgtgcgctc tcctgttccg accctgccgc 4440

ttaccggata cctgtccgcc tttctccctt cgggaagcgt ggcgctttct catagctcac 4500

gctgtaggta tctcagttcg gtgtaggtcg ttcgctccaa gctgggctgt gtgcacgaac 4560

cccccgttca gcccgaccgc tgcgccttat ccggtaacta tcgtcttgag tccaacccgg 4620

taagacacga cttatcgcca ctggcagcag ccactggtaa caggattagc agagcgaggt 4680

atgtaggcgg tgctacagag ttcttgaagt ggtggcctaa ctacggctac actagaagaa 4740

cagtatttgg tatctgcgct ctgctgaagc cagttacctt cggaaaaaga gttggtagct 4800

cttgatccgg caaacaaacc accgctggta gcggtggttt ttttgtttgc aagcagcaga 4860

ttacgcgcag aaaaaaagga tctcaagaag atcctttgat cttttctacg gggtctgacg 4920

ctcagtggaa cgaaaactca cgttaaggga ttttggtcat gagattatca aaaaggatct 4980

tcacctagat ccttttaaat taaaaatgaa gttttaaatc aatctaaagt atatatgagt 5040

aaacttggtc tgacagttac caatgcttaa tcagtgaggc acctatctca gcgatctgtc 5100

tatttcgttc atccatagtt gcctgactcc ccgtcgtgta gataactacg atacgggagg 5160

gcttaccatc tggccccagt gctgcaatga taccgcgaga cccacgctca ccggctccag 5220

atttatcagc aataaaccag ccagccggaa gggccgagcg cagaagtggt cctgcaactt 5280

tatccgcctc catccagtct attaattgtt gccgggaagc tagagtaagt agttcgccag 5340

ttaatagttt gcgcaacgtt gttgccattg ctacaggcat cgtggtgtca cgctcgtcgt 5400

ttggtatggc ttcattcagc tccggttccc aacgatcaag gcgagttaca tgatccccca 5460

tgttgtgcaa aaaagcggtt agctccttcg gtcctccgat cgttgtcaga agtaagttgg 5520

ccgcagtgtt atcactcatg gttatggcag cactgcataa ttctcttact gtcatgccat 5580

ccgtaagatg cttttctgtg actggtgagt actcaaccaa gtcattctga gaatagtgta 5640

tgcggcgacc gagttgctct tgcccggcgt caatacggga taataccgcg ccacatagca 5700

gaactttaaa agtgctcatc attggaaaac gttcttcggg gcgaaaactc tcaaggatct 5760

taccgctgtt gagatccagt tcgatgtaac ccactcgtgc acccaactga tcttcagcat 5820

cttttacttt caccagcgtt tctgggtgag caaaaacagg aaggcaaaat gccgcaaaaa 5880

agggaataag ggcgacacgg aaatgttgaa tactcatact cttccttttt caatattatt 5940

gaagcattta tcagggttat tgtctcatga gcggatacat atttgaatgt atttagaaaa 6000

ataaacaaat aggggttccg cgcacatttc cccgaaaagt gccacctgac gtctaagaaa 6060

ccattattat catgacatta acctataaaa ataggcgtat cacgaggccc tttcgtc 6117

<210> 10
<211> 49
<212> DNA
<213> Artificial

<220>
<223> Primer

<400> 10
caagaattcc tcgagtcagt ggtggtggtg gtggtggtta gcctccccc 49

<210> 11
<211> 34

<212>  DNA
<213>  Artificial

<220>
<223>  Primer

<400>  11
tacgtcgaca tggcttcgta cccctgccat cagc                                    34


<210>  12
<211>  35
<212>  DNA
<213>  Artificial

<220>
<223>  Primer

<400>  12
acgcgtcgac agagatatcg acaccatgga caggg                                   35


<210>  13
<211>  36
<212>  DNA
<213>  Artificial

<220>
<223>  Primer

<400>  13
gccttaatta atcagtagcc ctgcctcacc ctgttc                                  36


<210>  14
<211>  25
<212>  DNA
<213>  Artificial

<220>
<223>  Primer

<400>  14
taacgtgatg gatatattaa agtcg                                              25


<210>  15
<211>  24
<212>  DNA
<213>  Artificial

<220>
<223>  Primer

<400>  15
aacgacacaa catccatttt taag                                               24


<210>  16
<211>  42
<212>  DNA
<213>  Artificial

<220>
<223>  Primer

<400>  16
catacgcgat cagaattcat cgttgacatc tagtattgat ag                          42


<210>  17
<211>  46
<212>  DNA
<213>  Artificial

<220>
<223>  Primer

<400>  17
taaggcctct cgaggcgcgc cctatcaact acctataaaa ctttcc                      46


<210>  18
<211>  45
<212>  DNA
<213>  Artificial

<220>
<223>  Primer

<400>  18
gagaacctcg agttaattaa tgacttacat aaatgtctgg gatag                       45


<210>  19
<211>  34
<212>  DNA
<213>  Artificial

<220>
<223>  Primer

<400>  19
tctaggcctt gttaaaatac attctaatac ggtc                                   34


<210>  20
<211>  35
<212>  DNA
<213>  Artificial

<220>
<223>  Primer

<400>  20
aaatctcgag agaattaatc ccgctctatg gtcag                                  35


<210>  21
<211>  34
<212>  DNA
<213>  Artificial

<220>
<223>  Primer

<400>  21
gcgaagcttt tgttctatca actacctata aaac                                    34

<210>  22
<211>  25
<212>  DNA
<213>  Artificial

<220>
<223>  Primer

<400>  22
tgttaaaata cattctaata cggtc                                              25

<210>  23
<211>  24
<212>  DNA
<213>  Artificial

<220>
<223>  Primer

<400>  23
atcgttgaca tctagtattg atag                                               24